# EUROPÄISCHE PATENTSCHRIFT

(11) **EP 0 955 901 B1**
(45) Veröffentlichungstag und Bekanntmachung des Hinweises auf die Patenterteilung: **23.04.2003**
(21) Anmeldenummer: 97949934.0
(22) Anmeldetag: 17.11.1997
(51) Int. Cl.: A61B 17/00, A61B 17/08, A61F 13/02

(54) **DICHTER PUNKTIONSVERSCHLUSS**
TIGHT PUNCTURE SEAL
TAMPON D'OBTURATION ETANCHE POUR ORIFICE DE PONCTION

(30) Priorität: 16.11.1996 DE 19647496; 21.06.1997 DE 19726386
(43) Veröffentlichungstag der Anmeldung: 17.11.1999
(73) Patentinhaber: CAP Incorporated, Panama City (PA)
(72) Erfinder: Harren, Ernst-Diethelm, 52146 Würselen (DE); BANGERT, Christian, 52066 Aachen (DE)
(74) Vertreter: WALTHER, WALTHER & HINZ Patentanwälte
(86) Internationale Anmeldenummer: DE9702684
(87) Internationale Veröffentlichungsnummer: WO98022027

(56) Entgegenhaltungen:
- EP-A- 0 134 745
- WO-A-95/04511
- WO-A-96/05774
- DE-A- 4 418 910

## Beschreibung

Die vorliegende Erfindung betrifft einen Arterien-Punktionsverschluß zum Verschliessen eines eine Punktionsöffnung aufweisenden arteriellen Blutgefäßes im menschlichen oder tierischen Körper mittels Eigenblut, mit einer mit Überdruck beaufschfagbaren, im Bereich der Punktionsöffnung am Körper befestigbaren Druckkammer, wobei die Druckkammer in ihrem vom Körper abgewandten Bereich eine Haltewand aufweist.

Aus der DE-44 29 230, der WO 96/05774 oder der WO 97/06735 (nachveröffentlicht) sind Punktionsverschlüsse zum Verschließen eines eine Punktionsöffnung aufweisenden Blutgefäßes bekannt, deren Druckkammer solange mit dem aus dem Blutgefäß herausströmenden Blut gefüllt wird, bis sich in der Druckkammer der in dem Blutgefäß befindliche Blutdruck ausgebildet hat, so daß ein Druckgleichgewicht zwischen dem Blutgefäß und der Druckkammer herrscht. Durch dieses Druckgleichgewicht kommt die Blutung zum Stillstand. Der aus der DE-44 29 230 oder der WO 96/05774 bekannte Punktionsverschluß hat eine nahezu nicht dehnbare Haltewand, an deren Unterseite eine leicht dehnbare Druckwand, vorzugsweise aus Latex, angebracht ist.

Vor Beginn des therapeutischen oder diagnostischen Eingriffs wird der Punktionsverschluß im Bereich der zu erwartenden Punktierung des Blutgefäßes auf den menschlichen oder tierischen Körper aufgeklebt.

Anschließend wird die Kanüle der Injektionsspritze, des Katheters oder dergleichen durch die Druckkammer, insbesondere durch die Haltewand und durch die Druckwand hindurchgestoßen, um anschließend durch die Haut und das Gewebe des Patienten bis in das betreffende Blutgefäß zu gelangen, so daß nun die erforderliche Behandlungsmaßnahme durchgeführt werden kann.

Um beim Durchstechen der Kanüle die Gefahr des Ausstanzens von Materialpartikeichen aus der Haltewand auszuschließen ist vorgeschlagen worden, in der Haltewand und/oder der Druckwand vorgefertigte Öffnungen vorzusehen. Letzteres hat sich jedoch nicht als praktikabel erwiesen, da das Blut durch diese vorgefertigten Öffnungen austreten kann und beispielsweise eine der am Punktionsverschluß vorhandenen Klebeschichten schwächt, so daß das Blut unkontrolliert austreten kann.

Nach Beendigung der Behandlung wird die Kanüle aus dem Körper des Patienten und dem Punktionsverschluß herausgezogen, wobei der Punktionsverschluß am Körper kleben bleibt. Sodann wird die in der Haltewand befindliche Öffnung durch eine auf der Haltewand angebrachte, mit einem Kleber ausgerüstete Verschlußlasche geschlossen, siehe DE 44 29 230, WO 96/05774 oder WO 97/06735. Hierbei kann es gelegentlich vorkommen, daß einzelne Bluttropfen aus der Druckkammer und/oder der Kanüle entweichen, bevor die Verschlußlasche die Öffnung abdichtet. Diese Bluttropfen sind unhygienisch und stellen eine Infektionsgefahr für das behandelnde Personal dar. Außerdem wird durch diese Blutfropfen auch der Kleber derart geschwächt, daß ein druckdichtes Aufkleben der Verschlußlasche auf der Haltewand nicht mehr gewährleistet möglich ist.

Davon ausgehend liegt der vorliegenden Erfindung die Aufgabe zugrunde, einen Punktionsverschluß zu schaffen, dessen Druckkammer nach dem Entfernen der Kanüle zuverlässig verschlossen werden kann.

Als technische Lösung dieser Aufgabe wird erfindungsgemäß vorgeschlagen, den Punktionsverschluß der eingangs genannten Art durch ein mindestens 1 mm dickes Verschlußelement aus einem Material mit elastischer Rückstellkraft weiterzubilden, das in einem für den Einstich der Kanüle vorgesehenen Bereich der Haltewand angebracht ist, wobei das Verschlußelement aus einem Material mit einer elastischen Rückstellkraft, vorzugsweise zumindest teilweise aus Naturkautschuk, Kunstkautschuk, Gummi, Latex, Silikon, Flüssigsilikon, Hydrogel, polymerem Kunststoff oder einer Kombination einiger der vorgenannten Materialien gebildet ist.

Ein nach dieser technischen Lehre ausgeführter Punktionsverschluß hat den Vorteil, daß die Kanüle beim Eintritt in das Verschlußelement das Material mit elastischer Rückstellkraft auseinanderschiebt, beziehungsweise verdrängt, so daß das Ausstanzen beziehungsweise Heraustrennen von Materialpartikeln zuverlässig vermieden wird. Darüber hinaus wird die beim Auseinanderschieben des Materials aufgewendete Energie in diesem Material mit elastischer Rückstellkraft gespeichert, so daß das Material nach dem Herausziehen der Kanüte wieder in seine ursprüngliche Position zurückstrebt und somit die durch das Einstechen der Kanüle hervorgerufene Öffnung wieder verschließt.

Außerdem bewirkt das durch die Kanüle auseinandergeschobene Material des Verschlußefementes eine zuverlässige Abdichtung der Punktionsstelle und schützt diese während des Eingriffs vor Umwelteinflüssen, da das Material mit der elastischen Rückstellkraft aufgrund seiner Rückstellkraft stets dicht an der Kanüle anliegt.

In einer bevorzugten Ausführungsform ist das vorzugsweise kugelsegmentförmig oder linsenartig ausgebildete Verschlußelement auf der Haltewand angebracht, insbesondere aufgeklebt. Die Befestigung des Verschlußelementes an der Haltewand hat den Vorteil, daß hierdurch die Haltewand verstärkt wird.

Wie bereits in der DE 44 29 230 oder der WO 96/05774 ausgeführt ist, weist ein vorteilhafter Punktionsverschluß eine wenig dehnbare oder starre Haltewand auf, um eine Ausdehnung der Druckkammer vom Körper des Patienten weg zu vermeiden. Diese wenig dehnbare Haltewand bewirkt, daß sich die Druckkammer hauptsächlich zum Körper hin ausdehnt, und daß das zwischen dem Punktionsverschluß und dem Blutgefäß liegende Gewebe zusammendrückt wird, so daß ein Abfließen des Blutes ins Gewebe verhindert wird.

Durch die Verstärkung der Haltewand mittels Aufbringung eines erfindungsgemäßen Verschlußelementes ist es nun möglich, die Haltewand an sich genauso wie die Druckwand aus einem dehnbaren Material zu bilden, denn die an sich dehnbare Haltewand wird durch das daran befestigte Verschlußelement derart unelastisch, daß eine Ausdehnung der Druckkammer vom Körper weg in ausreichender Weise verhindert wird. Die Ausbildung der Haltewand an sich und der Druckwand aus ein und demselben dehnbaren Material hat weiterhin den Vorteil, daß die Haltewand und die Druckwand nunmehr linien- oder flächenhaft miteinander verschweißt werden können, so daß ein derart gefertigter Punktionsverschluß kostengünstig herstellbar ist, und daß die nunmehr stoffschlüssig miteinander verbundenen Wände der Druckkammer den im menschlichen und tierischen Körpern herrschenden Blutdruck zuverlässig standhalten.

Es hat sich herausgestellt, daß ein Punktionsverschluß mit einer Halte- und Druckwand aus Polyetherurethan gleicher Dicke (Stärke) mit einem auf der Haltewand angebrachten Verschlußelement bei der Anwendung unter Druck eine ausreichende Ausdehnung zum Körper hin aufweist. Trotzdem kann es vorteilhaft sein, die Haltewand dicker als die Druckwand auszuführen, da hierdurch die Ausdehnung der Haltewand weiter eingedämmt wird.

Das Abdecken des Verschlußelementes mit einer wenig dehnbaren oder starren Deckschicht, vorzugsweise aus Polyester oder Polyetherurethan hat den Vorteil, daß hierdurch die Ausdehnung der Haltewand vom Körper weg weiter erschwert wird, insbesondere dann, wenn sich die Deckschicht über den für den Einstich der Kanüle vorgesehenen Bereich hinaus erstreckt. Der hiermit erreichte sandwichartige Aufbau mit Haltewand-Verschlußelement-Deckschicht verhindert eine zu große Ausdehnung der Haltewand vom Körper weg zuverlässig.

Ein weiterer Vorteil der Deckschicht besteht darin, daß hierdurch das Verschlußelement gegen Verschmutzung und/oder Beschädigungen geschützt ist.

in einer bevorzugten Weiterbildung ist die Deckschicht mit Druck bzw. Vorspannung auf das insbesondere linsenförmig ausgebildete Verschlußelement aufgeklebt, so daß das Verschlußelement einem gewissen Druck, beziehungsweise einer gewissen Vorspannung ausgesetzt ist. Hierdurch wird die Rückstellwirkung des Materials mit elastischer Rückstellkraft des Verschlußelementes verstärkt, da das Verschlußelement bereits aufgrund des vorhandenen Druckes bestrebt ist, die Einstichöffnung der Kanüle zu verschließen, sobald diese aus dem Punktionsverschluß herausgezogen ist. Um diesen Effekt noch zu verstärken, ist es vorteilhaft, die Deckschicht aus einem nicht oder nur schwer dehnbaren Material wie beispielsweise einer Polyesterfolie oder einer Folie auf Polyesterbasis zu fertigen, damit der am Verschlußelement aufgebaute Druck erhalten bleibt.

Das Erstrecken der Deckschicht bis zum Rand des Punktionsverschlusses oder darüber hinaus hat den Vorteil, daß hierdurch der Punktionsverschluß mit dem Finger leicht greifbar ist, da er so in seiner Gesamtheit eine für Anwender ertastbare Stabilität aufweist, und daß eine über den Druckkammerrand hinaus vergrößerte Klebefläche zur Befestigung des Punktionsverschlusses auf der Haut des Patienten zur Verfügung steht.

in einer bevorzugten Ausführungsform ist der Punktionsverschluß zumindest teilweise, vorzugsweise im Bereich außerhalb der Druckkammer atmungsaktiv ausgebildet. Dies hat den Vorteil, daß der auf der Haut aufgeklebte Punktionsverschluß Schweiß oder andere Dämpfe durchläßt, so daß sich unter dem Punktionsverschluß keine Nässe ansammelt, die den Kleber angreift, und daß der Punktionsverschluß für den Patienten angenehm zu tragen ist.

in einer besonders bevorzugten Ausführungsform sind sowohl die Haltewand, als auch die Druckwand aus demselben dehnbaren Material ausgeführt, wobei die Haltewand dicker als die Druckwand ist. Durch die unterschiedliche Materialdicke sind die Haltewand und die Druckwand unterschiedlich dehnbar. Es hat sich gezeigt, daß diese unterschiedliche Dehnbarkeit zwischen Haltewand und Druckwand ausreicht, um die durch den Druck innerhalb der Druckkammer entstehenden Kräfte in ausreichender Form auf das Gewebe zu lenken, so daß sich die Punktionsöffnung verschließt. Dies gilt insbesondere auch dann, wenn die Haltewand und die Druckwand aus einer dehnbaren Polyetherurethanfolie, einer Polyurethanfolie oder einer Polypropylenfolie gefertigt sind, wobei die Haltewand eine Dicke von 30 µm bis 300 µm und die Druckwand eine Dicke von 5 µm bis 100 µm aufweist. Beim Einsatz des Punktionsverschlußes in der Dialyse hat die Haltewand eine Dicke von vorzugsweise 40 µm und die Druckwand eine Dicke von vorzugsweise 25 µm. Beim Einsatz des Punktionsverschlußes in der Kardiologie hat die Haltewand eine Dicke von vorzugsweise 100 µm und die Druckwand eine Dicke von vorzugsweise 60 µm. Außerdem hat sich herausgestellt, daß beim Einsatz von Polyetherurethan keine Partikel von der Kanüle ausgestanzt werden, die in die Blutbahn gelangen könnten.

Weitere Vorteile einer Halte- und Druckwand aus Polyetherurethan, Polyether oder Polypropylen bestehen darin, daß diese Materialien atmungsaktiv und transparent sind, so daß ein derartig aufgebauter Punktionsverschluß auf der Haut angenehm zu tragen ist, und daß auch bei aufgeklebtem Punktionsverschluß die zu punktierende Stelle auf dem Körper des Patienten sichtbar bleibt. Dieser bevorzugte Punktionsverschluß wird beispielsweise bei der Blutwäsche bei Dialysepatienten eingesetzt.

in einer alternativen Ausführungsform des erfindungsgemäßen Punktionsverschlusses ist die gesamte Haltewand aus einem Material mit einer elastischen Rückstellkraft gebildet, wobei die Haltewand entweder eine einheitliche Dicke aufweist oder aber im Einstichbereich eine entsprechende Verdickung hat. An die Unterseite einer derartigen Haltewand ist dann eine im Vergleich zur Haltewand dehnbare Druckwand angeklebt.

Sowohl bei einer aus elastischem Material hergestellten Haltewand als auch beim Verschlußelement ist darauf zu achten, daß die verwendete Materialdicke ausreicht, um die durch die Kanüle geschaffene Öffnung wieder zu verschließen. Bei entsprechenden Versuchen mit einem Verschlußelement aus Silikon hat sich herausgestellt, daß eine Materialdicke von zirka 4 mm ausreicht, um die von einer im Bereich der Dialyse verwendeten Kanüte mit einem Außendurchmesser von 1,8 mm geschaffene Öffnung zuverlässig zu verschließen. Beim Einsatz des erfindungsgemäßen Punktionsverschlusses im Bereich der Kardiologie treten sehr viel größere Öffnungsdurchmesser auf, so daß hier eine Materialstärke von bis zu 25 mm vorteilhaft ist.

Da der Blutdruck in den Arterien in Abhängigkeit vom Puls (z. B. zwischen zwei Pulsschlägen zwischen einem Maximalwert und einem Minimalwert schwankt, fließt aufgrund des einmal in der Druckkammer aufgebauten Druckes wieder ein gewisser Anteil des Blutes aus der Druckkammer hinaus, sobald der Druck in der Arterie kurzzeitig sinkt. Um dies zu verhindern, ist es vorteilhaft die Druckwand aus einer 0,2 mm bis 3 mm, vorzugsweise 0,5 mm bis 1 mm, dicken Schicht aus einem Material mit elastischer Rückstellkraft zu bilden oder aber innerhalb der Druckkammer an der Druckwand eine 0,2 mm bis 3 mm, vorzugsweise 0,5 mm bis 1 mm, dicke Schließschicht aus einem Material mit elastischer Rückstellkraft anzubringen.

In beiden Fällen strömt das Blut aus dem Blutgefäß in die Druckkammer und bewirkt zunächst, daß die Öffnung durchgängig bleibt, da die Rückstellkraft des Materials nicht ausreicht, die Öffnung vollständig zu verschließen.
Wenn in der Druckkammer ein gewisser Druck ausgebildet ist, wird hierdurch das Schließelement bzw. die Druckwand teilweise zusammengedrückt, so daß dies in Kombination mit der Rückstellkraft des Materials ausreicht, um die Öffnung in der Druckwand zu schließen. Folglich verkürzt sich die Füllzeit der Druckkammer durch die wie ein Rückschlagventil wirkende Schließschicht, beziehungsweise Druckwand, und es ist innerhalb kürzester Zeit ein höherer Maximaldruck in der Druckkammer erzielbar. Beides führt zu einem höheren Nutzwert des erfindungsgemäßen Punktionsverschlusses.

In einer anderen, bevorzugten Ausführungsform ist die Haltewand aus einem Material mit elastischer Rückstellkraft gebildet, welches mit Fasern verstärkt ist. Diese unelastischen Fasern können zum Beispiel kreuzweise verlegte Langfasern oder ein Netz sein.

Dies hat den Vorteil, daß die Haltewand einstückig mit dem Verschlußelement aus Kautschuk, Gummi, Latex, Hydrogel, (Flüssigkeits-) Silikon, polymeren Kunststoff oder dergleichen gefertigt, insbesondere gegossen, werden kann, aber durch die unelastischen Fasern nur wenig dehnbar ist, so daß der sich in der Druckkammer ausbildende Druck lediglich die Druckwand, nicht aber die Haltewand ausdehnt.

in noch einer weiteren bevorzugten Ausführungsform sind die Haltewand und die Druckwand durch einen Silikonkleber oder einen synthetischen Kautschuk miteinander verklebt. Das Verkleben von Haltewand und Druckwand wird bevorzugt dann eingesetzt, wenn die Haltewand und die Druckwand aus unterschiedlichen Materialien gefertigt sind. Problematisch ist hierbei jedoch, daß die Klebeschicht aufgrund der diracartigen Belastung stark auf Schälung beansprucht wird und in der Regel keine ausreichende Haftkraft entfaltet. Bei der Verwendung von Silikonkleber oder einem synthetischen Kautschuk, insbesondere wenn die Klebeschicht 0,1 mm bis 1 mm dick ist, wird die Linienbelastung über die Schichtdicke in eine Flächenbelastung überführt, so daß es dem Klebstoff nun sehr viel einfacher möglich ist, die auftretenden Kräfte zu beherrschen und die beiden Wände zusammenzuhalten.

Da der Haftkleber im Bereich der Punktionsöffnung nur eine kraftlos dichtende Funktion hat, ist in einer bevorzugten Weiterbildung im Mittelbereich der Druckkammer ein Haftkleber aufgetragen, der eine geringere Klebekraft aufweist, als der übrige Haftkleber, bzw. ist der Mittelbereich der Druckkammer klebstofffrei gehalten. Dies hat den Vorteil, daß der Punktionsverschluß leichter wieder vom Körper des Patienten abgezogen werden kann, ohne daß die geschlossene Punktionsstelle starken Kräften ausgesetzt ist.

Weitere Vorteile des erfindungsgemäßen Punktionsverschlusses ergeben sich aus der Beschreibung und aus der beigefügten Zeichnung. Ebenso können die vorstehend genannten und die noch weiter ausgeführten Merkmale erfindungsgemäß jeweils einzeln oder in beliebigen Kombinationen miteinander verwendet werden. Die erwähnten Ausführungsformen sind nicht als abschließende Aufzählung zu verstehen, sondern haben vielmehr beispielhaften Charakter. Die Erfindung ist in der Zeichnung dargestellt und wird anhand von Ausführungsbeispielen näher erläutert. Es zeigen:
- Fig. 1: eine geschnittene Seitenansicht eines erfindungsgemäßen Punktionsverschlusses mit integriertem Arzneimittelträger;
- Fig. 2: eine geschnittene Seitenansicht eines erfindungsgemäßen Punktionsverschlusses mit Deckschicht;
- Fig. 3: eine geschnittene Seitenansicht eines erfindungsgemäßen Punktionsverschlusses mit verlängerter Deckschicht;
- Fig. 4: eine geschnittene Seintenansicht eines erfindungsgemäßen Punktionsverschlußes mit einer in der Druckkammer integrierten Schließschicht;
- Fig. 5: eine geschnittene Seitenansicht eines erfindungsgemäßen Punktionsverschlusses mit in der Haltewand integriertem Verschlußelement.

Die einzelnen Figuren der Zeichnung zeigen den erfindungsgemäßen Gegenstand, teilweise stark überproportional vergrößert dargestellt, damit sein Aufbau besser gezeigt werden kann.

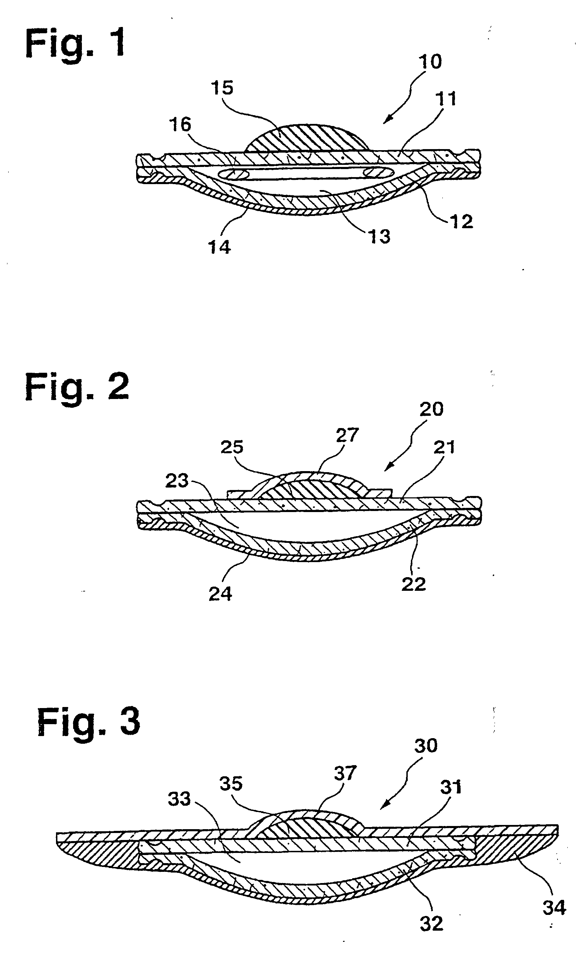

Die in den Fig. 1 bis 4 dargestellten vier verschiedenen Ausführungsformen haben alle den gleichen Kernaufbau, denn alle diese Punktionsverschlüsse 10, 20, 30, 40 verfügen über eine relativ gering dehnbare Haltewand 11, 21, 31, 41 aus einer 40 µm dicken Polyetherurethanfolie, die mit einer Druckwand 12, 22, 32, 42 aus einer 25 µm dicken Polyetherurethanfolie linien- oder flächenhaft verschweißt ist, so daß zwischen der Haltewand 11, 21, 31, 41 und der Druckwand 12, 22, 32, 42 eine Druckkammer 13, 23, 33, 43 zur Aufnahme des aus dem Blutgefäß ausströmenden Blutes ausgebildet ist. Auf der dem Körper zugewandten Seite des Punktionsverschlusses 10, 20, 30, 40 ist ein hautverträglicher, biokompatibler Klebstoff 14, 24, 34, 44, vorzugsweise auf Acrylat- oder Silikonbasis aufgetragen, mit dem der Punktionsverschluß 10, 20, 30, 40 auf der Haut des Patienten festgeklebt werden kann. Um diese Klebstoffschicht transportabel und funktionstüchtig zu halten, ist eine hier nicht dargestellte Schutzfolie aufzubringen.

Auf der Oberseite der Haltewand 11, 21, 31, 41, das heißt, auf der dem Körper abgewandten Seite des Punktionsverschlusses 10, 20, 30, 40 ist auf die Haltewand 11, 21, 31, 41 ein Verschlußelement 15, 25, 35, 45 aufgeklebt oder aufvulkanisiert, welches vorzugsweise mittig über der jeweiligen Druckkammer 13, 23, 33, 43 angeordnet ist. Dieses Verschlußelement 15, 25, 35, 45 besteht aus Silikon und verstärkt zum Einen die Haltewand 11, 21, 31, 41 und verschließt zum Anderen die beim Einstechen der Kanüle verursachte Öffnung, nachdem die hier nicht dargestellte Kanüle wieder entfernt wurde. In den hier dargestellten Ausführungsformen gemäß den Figuren 1 bis 4 ist das Verschlußelement 15, 25, 35, 45 kreissegmentförmig ausgebildet, das heißt, das Verschlußelement 15, 25, 35, 45 ist auf seiner Unterseite eben und auf seiner Oberseite gekrümmt ausgeführt. Mit anderen Worten ausgedrückt könnte man auch sagen, daß das Verschlußelement 15, 25, 35, 45 halblinsenförmig ausgebildet ist.

Diese Basisversion eines erfindungsgemäßen Punktionsverschlusses hat zwar eine Haltewand 11, 21, 31, 41 und eine Druckwand 12, 22, 32, 42 aus ein und demselben Material, jedoch ist die Haltewand 11, 21, 31, 41 dicker ausgeführt, so daß diese weniger dehnbar ist, als die Druckwand 12, 22, 32, 42. Dieses an sich gut dehnbare Polyetherurethan wird im Bereich des zu erwartenden Einstiches der Kanüle und auch darüber hinaus durch das kugelsegmentförmig ausgebildete Verschlußelement 15, 25, 35, 45 aus Silikon zusätzlich verstärkt, so daß eine Ausdehnung der Haltewand 11, 21, 31, 41 bei gefüllter Druckkammer 13, 23, 33, 43 nur in geringem Maße erfolgt, so daß die Hauptausdehnung der Druckkammer 13, 23, 33, 43 über die Druckwand 12, 22, 32, 42 in Richtung des Körpers des Patienten erfolgt, so daß ein Entweichen des Blutes in das Gewebe zuverlässig vermieden wird, da dieses unter Druck steht.

Durch die Verwendung gleichartiger Materialien als Druck- 12, 22, 32, 42 und Haltewand 11, 21, 31, 41 ist ein einfaches Verschweißen dieser beiden Wände möglich, so daß eine ausreichend druckdichte Druckkammer 13, 23, 33, 43 kostengünstig hergestellt werden kann.

In der in Figur 1 dargestellten Ausführungsform des erfindungsgemäßen Punktionsverschlusses 10 ist in der Druckkammer 12 ein Arzneimittelträger aus Mull oder einer Gewebematrix integriert. Dieser ringförmig ausgebildete Arzneimittelträger 16 kann mit einem Hämostatikum zur schnelleren Blutgerinnung oder mit einem anderen Arzneimittel getränkt sein.

In der in Figur 2 dargestellten Ausführungsform ist der Punktionsverschluß 20 auf seiner Oberseite mit einer aus einer 40 µm dicken Polyesterfolie gebildeten Deckschicht 27 versehen, die das Verschlußelement 25 vollständig abdeckt. Diese Deckschicht 27 liegt so eng am Verschlußelement 25 an, daß dieses zumindest leicht unter Druck steht. Dabei ist die Deckseicht 27 aufgrund der Klebewirkung des Silikons des Verschlußelementes 25 aufgeklebt. Durch diesen Druck auf das Verschlußelement 25 wird die Rückstellkraft des Silikons verstärkt, da nun zusätzlich von außen wirkende Kräfte auf die durch die Kanüle verursachte Öffnung einwirken, um diese zu verschließen. Verstärkt wird dieser Effekt noch dadurch, daß bei gefüllter Druckkammer 27 das Verschlußelement 25 an der Haltewand 21 tangential zu dieser zusammengedrückt wird und so den den Durchstich schließenden Druck auf das Verschlußelement 25 weiter erhöht.

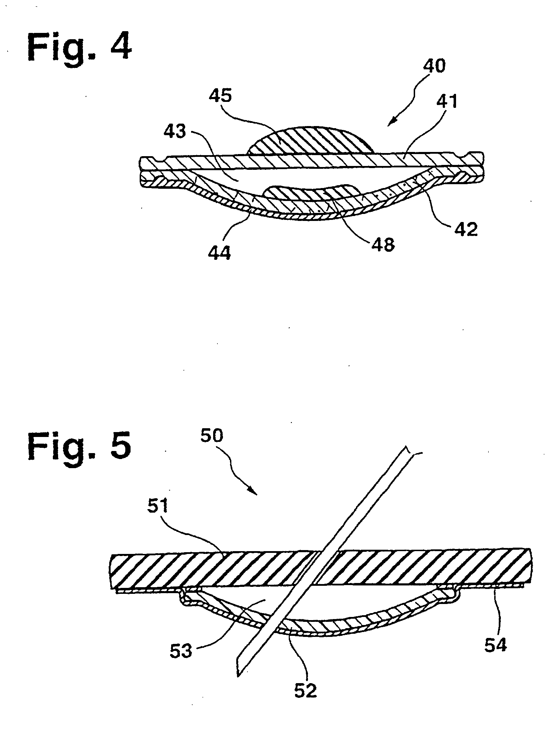

In der in Figur 3 dargesteliten Ausführungsform ist die Deckschicht 37 sehr viel großflächiger als das Verschlußelement 35 selbst ausgebildet und erstreckt sich bis über den Rand der Haltewand 31, so daß die Deckschicht 37 deutlich über die Haltewand 31 hinausragt. In dem überragenden Bereich der Deckschicht 37 ist körperseitig ebenfalls Klebstoff 34 angebracht, damit der Punktionsverschluß 30 zuverlässig am Körper des Patienten angeklebt werden kann.

In der in Figur 4 dargestellten Ausführungsform ist in der Druckkammer 43 des Punktionsverschlusses 40 eine Schließschicht 48 aus einem Material mit elastischer Rückstellkraft, vorzugsweise Silikon, ingegriert. Diese Schließschicht 48 sitzt auf der Innenseite der Druckwand 42 und ist in dem Bereich angeordnet, in dem der Durchstich der Kanüle zu erwarten ist. Diese Schließschicht 48 wirkt ähnlich wie ein Rückschlagventil und verhindert das Ausfließen von Blut aus der Druckkammer 43, während einem kurzzeitigen Druckabfall im Blutgefäß.

In Figur 5 ist eine alternative Ausführungsform eines erfindungsgemäßen Punktionsverschlusses 50 gezeigt, bei dem die Haltewand 51 aus einer dicken (Natur-) Kautschuk-, Latex- oder Silikonschicht besteht, an deren Unterseite eine leicht dehnbare Druckwand 52 aus einer 25 µm dicken Polyetherurethanfolie angeklebt ist, so daß sich zwischen der Haltewand 51 und der Druckwand 52 eine Druckkammer 53 ausbildet. Auch in dieser Ausführungsform ist die Unterseite des Punktionsverschlusses 50 mit dem oben erwähnten Klebstoff 54 überzogen.

In einer alternativen Ausführungsform kann die Deckwand genauso wie die Haltewand 51 aus (Natur-) Kautschuk, Latex oder Silikon gebildet sein. Im Gegensatz zur Haltewand 51 ist die Druckwand 52 dann aber nur 0,5 mm dick.

Sämtliche oben beschriebenen Punktionsverschlüsse 10, 20, 30, 40, 50 werden beispielsweise im Bereich der Dialyse oder Kardiologie eingesetzt.

Bei einem Einsatz in der Dialyse wird der erfindungsgemäße Punktionsverschluß am Unterarm im Bereich des Shunts eingesetzt, während der erfindungsgemäße Punktionsverschluß in der Kardiologie am Oberschenkel im Bereich der Femoralis eingesetzt wird. Beim Einsatz des Punktionsverschlusses in der Dialyse werden üblicherweise Kanülen mit einem Durchmesser von 1,8 mm eingesetzt, so daß zum Verschließen der durch die Kanüle verursachten Öffnung nach abgeschlossenem Eingriff ein Verschlußelement mit einer Dicke von 5 mm ausreichend ist. Beim Einsatz des Punktionsverschlusses im Bereich der Kardiologie werden mitunter sehr viel dickere Kanülen und/oder Katheder von bis zu 5 mm Durchmesser in das Blugefäß eingeführt, so daß hier je nach Anwendungsfall das Verschlußelement eine Dicke von bis zu 25 mm aufweist, um die Öffnung zuverlässig wieder zu verschließen.

In einer alternativen, hier nicht dargestellten Ausführungsform hat der Punktionsverschluß eine Halte- und eine Druckwand, die beide aus einer 150 µm dicken Polyetherurethan- oder Inzisionsfolie gebildet sind. Diese beiden 150 µm dicken Folien können mehrfach oder flächenhaft miteinander verschweißt oder vulkanisiert sein, so daß eine starke Verbindung zwischen den Folien entsteht, die auch den bei einer Anwendung in der Kardiologie in der Druckkammer auftretenden Druckkräften standhält.

Alle Punktionsverschlüsse 10, 20, 30, 40, 50 sind transparent ausgeführt, damit zumindest ungefähr das zu punktierende Blutgefäß sichtbar bleibt.

Sämtliche in dieser Anmeldung beschriebenen Elemente aus einem Material mit einer elastischen Rückstellkraft sind zumindest teilweise aus Gummi, Naturkautschuk, Kunstkautschuk, Latex, Silikon, Flüssigsilikon, Hydrogel, polymerem Kunststoff oder einer Kombination einiger der vorgenannten Materialien gebildet.

### Bezugszeichenliste:

- 10, 20, 30, 40, 50: Punktionsverschluß
- 11, 21, 31, 41, 51: Hattewand
- 12, 22, 32, 42, 52: Druckwand
- 13, 23, 33, 43, 53: Druckkammer
- 14, 24, 34, 44, 54: Klebstoff
- 15, 25, 35, 45: Verschlußelement
- 16: Arzneimittelträger
- 27, 37, 47: Deckschicht
- 48: Schließschicht

## Patentansprüche

1. Arterien-Punktionsverschluß zum Verschließen eines eine Punktionsöffnung aufweisenden arteriellen Blutgefäßes im menschlichen oder tierischen Körper mittels Eigenblut, mit einer mit Überdruck beaufschlagbaren, im Bereich der Punktionsöffnung am Körper befestigbaren Druckkammer (13, 23, 33, 43, 53), wobei die Druckkammer (13, 23, 33, 43, 53) in ihrem vom Körper abgewandten Bereich eine Haltewand (11, 21, 31, 41, 51) und ein Verschlußelement (15, 25, 35, 35) aufweist, **gekennzeichnet dadurch, daß** das Verschlußelement mindestens 1 mm dick ist, aus einem Material mit elastischer Rückstellkraft besteht, und in einem für den Einstich der Kanüle vorgesehenen Bereich der Haltewand (11, 21, 31, 41, 51) angebracht ist.

2. Punktionsverschluß nach Anspruch 1,
**dadurch gekennzeichnet,**
**daß** das Verschlußelement (15, 25, 35, 45) aus einem Material mit einer elastischen Rückstellkraft zumindest teilweise aus Gummi, Naturkautschuk, Kunstkautschuk, Latex, Silikon, Flüssigsilikon, Hydrogel, polymerem Kunststoff oder einer Kombination einiger der vorgenannten Materialien gebildet ist.

3. Punktionsverschluß nach einem der vorangehenden Ansprüche,
**dadurch gekennzeichnet,**
**daß** das Verschlußelement (15, 25, 35, 45) auf der Haltewand (11, 21, 31, 41) angebracht, insbesondere aufgeklebt ist.

4. Punktionsverschluß nach einem der vorangehenden Ansprüche,
**dadurch gekennzeichnet,**
**daß** das Verschlußelement (15, 25, 35, 45) kugelsegmentförmig ausgebildet ist.

5. Punktionsverschluß nach einem der vorangehenden Ansprüche,
**dadurch gekennzeichnet,**
**daß** über dem Verschlußelement (15, 25, 35, 45) eine wenig dehnbare oder starre zwischen 10 µm und 100 µm dicke Deckschicht (27, 37, 47), vorzugsweise aus Polyester oder Polyetherurethan, angeordnet ist.

6. Punktionsverschluß nach Anspruch 5,
**dadurch gekennzeichnet,**
**daß** sich die Deckschicht (27, 37, 47) über den für den Einstich der Kanüle vorgesehenen Bereich hinaus erstreckt.

7. Punktionsverschluß nach einem der Ansprüche 5 oder 6,
**dadurch gekennzeichnet,**
**daß** die Deckschicht (27, 37, 47) derart am Punktionsverschluß (20, 30, 40) befestigt ist, daß das Verschlußelement (25, 35, 45) dauerhaft unter Druck steht, bzw. vorgespannt ist.

8. Punktionsverschluß nach einem der vorangehenden Ansprüche,
**dadurch gekennzeichnet,**
**daß** die Druckkammer (13, 23, 33, 43, 53) in ihrem der Punktionsöffnung zugewandten Bereich eine dehnbare Druckwand (12, 22, 32, 42, 52) aufweist.

9. Punktionsverschluß nach Anspruch 8,
**dadurch gekennzeichnet,**
**daß** sowohl die Haltewand (11, 21, 31, 41), als auch die Druckwand (12, 22, 32, 42) aus dem selben dehnbaren Material sind.

10. Punktionsverschluß nach Anspruch 9,
**dadurch gekennzeichnet,**
**daß** die Haltewand (11, 21, 31, 41, 51) dicker als die Druckwand (12, 22, 32, 42, 52) ausgeführt ist.

11. Punktionsverschluß nach einem der Ansprüche 9 oder 10,
**dadurch gekennzeichnet,**
**daß** die Haltewand (11, 21, 31, 41) mit der Druckwand (12, 22, 32, 42) linien- und/oder flächenhaft verschweißt, insbesondere thermooder ultraschallverschweißt ist.

12. Punktionsverschluß nach einem der Ansprüche 9 bis 11,
**dadurch gekennzeichnet,**
**daß** die Haltewand (11, 21, 31, 41) aus einer 30 µm bis 100 µm, vorzugsweise 40 µm dicken Polyetherurethan-, Polyether- oder Polypropylenfolie und/oder die Druckwand (12, 22, 32, 42) aus einer 5 µm bis 50 µm, vorzugsweise 25 µm dicken Polyetherurethan-, Polyetheroder Polypropylenfolie gebildet ist.

13. Punktionsverschluß nach einem der Ansprüche 8 bis 12,
**dadurch gekennzeichnet,**
**daß** die Druckwand (42) innenseitig in einem für den Einstich der Kanüle vorgesehenen Bereich mit einer 0,2 mm bis 3 mm, vorzugsweise 0,5 mm bis 1 mm, dicken Schließschicht (48) aus einem Material mit elastischer Rückstellkraft ausgestattet ist.

14. Punktionsverschluß nach einem der Ansprüche 9 bis 11,
**dadurch gekennzeichnet,**
**daß** die Haltewand (51) aus einer 1 mm bis 25 mm, vorzugsweise 5 mm bis 10 mm dicken Kautschuk-, Latex- oder Silikonschicht und/oder die Druckwand aus einer 0,2 mm bis 3 mm, vorzugsweise 0,5 mm bis 1 mm, dicken Kautschuk-, Latex- oder Silikonschicht gebildet ist.

15. Punktionsverschluß nach Anspruch 14,
**dadurch gekennzeichnet,**
**daß** die Haltewand und/oder die Druckwand mittels im Material eingelassener Fasern verstärkt ist.

16. Punktionsverschluß nach einem der vorangehenden Ansprüche,
**dadurch gekennzeichnet,**
**daß** der Punktionsverschluß zumindest teilweise, vorzugsweise im Bereich außerhalb der Druckkammer (13, 23, 33, 43, 53) atmungsaktiv ausgebildet ist.

17. Punktionsverschluß nach einem der vorhergehenden Ansprüche,
**dadurch gekennzeichnet,**
**daß** der Punktionsverschluß zumindest im für den Einstich der Kanüle vorgesehenen Bereich, vorzugsweise im gesamten Bereich der Druckkammer (13, 23, 33, 43, 53), transparent ausgeführt ist.

## Claims

1. Arterial puncture closure for closing a punctured blood vessel in a human or animal body by means of autologous blood, said puncture closure having a pressure chamber (13, 23, 33, 43, 53) to which excess pressure can be applied and which can be fastened onto the body in the vicinity of the puncture, the part of the pressure chamber (13, 23, 33, 43, 53) that is turned away from the body being provided with a retaining wall (11, 21, 31, 41, 51) and with a closing element (15, 25, 35, 45),
**characterized in that** said closing element is at least 1 mm thick, is made of a material having an elastic restoring force and is arranged in an area of the retaining wall (11, 21, 31, 41, 51) in which the puncture by the cannula is planned.

2. Puncture closure according to claim 1,
**characterized in that** the closing element (15, 25, 35, 45), which is made of a material having an elastic restoring force, is at least partially made of natural caoutchouc, synthetic caoutchouc, rubber, latex, silicone, liquid silicone, hydrogel, polymeric plastic or a combination of at least some of the listed materials.

3. Puncture closure according to one of the previous claims,
**characterized in that** the closing element (15, 25, 35, 45) is arranged, more particularly glued, on the retaining wall (11, 21, 31, 41),

4. Puncture closure according to one of the previous claims,
**characterized in that** the closing element (15, 25, 35, 45) is designed as a spherical segment.

5. Puncture closure according to one of the previous claims,
**characterized in that** an outer layer (27, 37, 47) is disposed on the closing element (15, 25, 35, 45), said outer layer (27, 37, 47) being preferably made of polyester or polyetherurethane, being hardly extensible or rigid and having a thickness of between 10 µm and 100 µm.

6. Puncture closure according to claim 5,
**characterized in that** the outer layer (27, 37, 47) is extending beyond the area in which the puncture by the cannula is planned.

7. Puncture closure according to one of the claims 5 or 6,
**characterized in that** the outer layer (27, 37, 47) is fastened to the puncture closure (20, 30, 40) in such a way that the closing element (25, 35, 45) is constantly under pressure or pretension.

8. Puncture closure according to one of the previous claims,
**characterized in that** the pressure chamber (13, 23, 33, 43, 53) is provided, in its area facing the puncture opening, with an extensible pressure wall (12, 22, 32, 42, 52).

9. Puncture closure according to claim 8,
**characterized in that** the retaining wall (11, 21, 31, 41) as well as the pressure wall (12, 22, 32, 42) are made of the same extensible material.

10. Puncture closure according to claim 9,
**characterized in that** the retaining wall (11, 21, 31, 41, 51) is thicker than the pressure wall (12, 22, 32, 42, 52).

11. Puncture closure according to one of the claims 9 or 10,
**characterized in that** the retaining wall (11, 21, 31, 41, 51) is welded in lines or over the surface thereof to the pressure wall (12, 22, 32, 42) and is particularly heat welded or ultrasonic welded.

12. Puncture closure according to one of the claims 9 to 11,
**characterized in that** the retaining wall (11, 21, 31, 41) is made of a foil of polyetherurethane, polyether or polypropylene having a thickness of between 30 µm and 100 µm, preferably of 40 µem, and/or that the pressure wall (12, 22, 32, 42) is made of a foil of polyetherurethan, polyether or polypropylene having a thickness of between 5 µm and 50 µm, preferably of 25 µm.

13. Puncture closure according to one of the claims 8 to 12,
**characterized in that** a closing layer (48) made of a material with elastic restoring force and having a thickness of between 0,2 mm to 3 mm, preferably of between 0,5 mm to 1 mm, is arranged on the inner side of the pressure wall (42) in the area in which the puncture by the cannula is planned.

14. Puncture closure according to one of the claims 9 to 11,
**characterized in that** the retaining wall (51) is made of a layer of caoutchouc, latex or silicone having a thickness of between 1 mm and 25 mm, preferably of between 5 mm to 10 mm and/or that the pressure wall is made of a layer of caoutchouc, latex or silicone having a thickness of between 0,2 mm and 3 mm, preferably of between 0,5 mm and 1 mm.

15. Puncture closure according to claim 14,
**characterized in that** the retaining wall and/or the pressure wall are reinforced by fibres inserted in the material.

16. Puncture closure according to one of the previous claims,
**characterized in that** the puncture closure is at least partially air breathable, preferably in the area outside the pressure chamber (13, 23, 33, 43, 53).

17. Puncture closure according to one of the previous claims,
**characterized in that** the puncture closure is transparent, at least in the area where the puncture by the cannula is planned, preferably in the whole area of the pressure chamber (13, 23, 33, 43, 53).

## Revendications

1. Pansement occlusif pour ponction artérielle destiné à refermer une artère ponctionnée dans le corps humain ou animal au moyen du propre sang du patient avec une chambre de pression (13, 23, 33, 43, 53) susceptible d'être mise en surpression et destinée à être fixée sur le corps à l'endroit de l'orifice de ponction, la chambre de pression (13, 23, 33, 43, 53) comportant une paroi de retenue (11, 21, 31, 41, 51) et un élément de fermeture (15, 25, 35, 45) dans sa partie détournée du corps,
**caractérisé en ce que**
l'élément de fermeture présente une épaisseur d'au moins 1 mm, est réalisé dans un matériau doté d'une force de rappel élastique et est installé dans la paroi de retenue (11, 21, 31, 41, 51) au niveau d'une zone d'insertion prévue de la canule.

2. Pansement occlusif pour ponction selon la revendication 1,
**caractérisé en ce que**
l'élément de fermeture (15, 25, 35, 45) en un matériau doté d'une force de rappel élastique, est réalisé de préférence du moins en partie en caoutchouc naturel, en caoutchouc synthétique, en gomme, latex, silicone, silicone liquide, hydrogel, matière plastique polymère ou en une combinaison de quelques-uns des matériaux précités.

3. Pansement occlusif pour ponction selon l'une quelconque des revendications précédentes,
**caractérisé en ce que**
l'élément de fermeture (15, 25, 35, 45) est monté, notamment collé, sur la paroi de retenue (11, 21, 31, 41).

4. Pansement occlusif pour ponction selon l'une quelconque des revendications précédentes,
**caractérisé en ce que**
l'élément de fermeture (15, 25, 35, 45) est réalisé sous forme de segment sphérique.

5. Pansement occlusif pour ponction selon l'une quelconque des revendications précédentes,
**caractérisé en ce qu'**
une couche de recouvrement (27, 37, 47) peu extensible ou rigide, d'une épaisseur comprise entre 10 µm et 100 µm et réalisée de préférence en polyester ou en polyéther-uréthane est disposée au-dessus de l'élément de fermeture (15, 25, 35, 45).

6. Pansement occlusif pour ponction selon la revendication 5,
**caractérisé en ce que**
la couche de recouvrement (27, 37, 47) s'étend au-delà de la zone prévue pour l'insertion de la canule.

7. Pansement occlusif pour ponction selon l'une quelconque des revendications 5 ou 6,
**caractérisé en ce que**
la couche de recouvrement (27, 37, 47) est fixée au pansement occlusif (20, 30, 40) de telle sorte que l'élément de fermeture (25, 35, 45) est comprimé ou précontraint en permanence.

8. Pansement occlusif pour ponction selon l'une quelconque des revendications précédentes,
**caractérisé en ce que**,
dans la zone tournée vers l'orifice de ponction, la chambre de pression (13, 23, 33, 43, 53) comporte une paroi de pression (12, 22, 32, 42, 52) extensible.

9. Pansement occlusif pour ponction selon la revendication 8,
**caractérisé en ce que**
la paroi de retenue (11, 21, 31, 41) et la paroi de pression (12, 22, 32, 42) sont toutes deux réalisées dans le même matériau extensible.

10. Pansement occlusif pour ponction selon la revendication 9,
**caractérisé en ce que**
la paroi de retenue (11, 21, 31, 41, 51) est plus épaisse que la paroi de pression (12, 22, 32, 42, 52).

11. Pansement occlusif pour ponction selon l'une quelconque des revendications 9 ou 10,
**caractérisé en ce que**
la paroi de retenue (11, 21, 31, 41, 51) est soudée le long de lignes et/ou de façon surfacique à la paroi de pression (12, 22, 32, 42), notamment par thermosoudage ou par soudage par ultrasons.

12. Pansement occlusif pour ponction selon l'une quelconque des revendications 9 à 11,
**caractérisé en ce que**
la paroi de retenue (11, 21, 31, 41) est réalisée dans une feuille de polyéther-uréthane, de polyéther ou de polypropylène d'une épaisseur comprise entre 30 µm et 100 µm, de préférence d'une épaisseur de 40 µm, et/ou que la paroi de pression (12, 22, 32, 42) est réalisée dans une feuille de polyéther-uréthane, polyéther ou polypropylène d'une épaisseur comprise entre 5 µm et 50 µm, de préférence d'une épaisseur de 25 µm.

13. Pansement occlusif pour ponction selon l'une quelconque des revendications 8 à 12,
**caractérisé en ce que**
la paroi de pression (42) est munie sur sa face interne, dans une zone prévue pour l'insertion de la canule, d'une couche d'obturation (48) d'une épaisseur comprise entre 0,2 mm et 3 mm, de préférence entre 0,5 mm et 1 mm, et réalisée dans un matériau doté d'une force de rappel élastique.

14. Pansement occlusif pour ponction selon l'une quelconque des revendications 9 à 11,
**caractérisé en ce que**
la paroi de retenue (51) est réalisée dans une couche de caoutchouc, de latex ou de silicone d'une épaisseur comprise entre 1 mm et 25 mm, de préférence entre 5 mm et 10 mm, et/ou que la paroi de pression est réalisée dans une couche de caoutchouc, de latex ou de silicone d'une épaisseur comprise entre 0,2 mm et 3 mm, de préférence entre 0,5 mm et 1 mm.

15. Pansement occlusif pour ponction selon la revendication 14,
**caractérisé en ce que**
la paroi de retenue et/ou la paroi de pression est renforcée par des fibres incorporées dans le matériau.

16. Pansement occlusif pour ponction selon l'une quelconque des revendications précédentes,
**caractérisé en ce que**
le pansement occlusif est réalisé respirant, du moins en partie et de préférence dans la zone située en dehors de la chambre de pression (13, 23, 33, 43, 53).

17. Pansement occlusif pour ponction selon l'une quelconque des revendications précédentes,
**caractérisé en ce que**
le pansement occlusif est réalisé transparent, du moins dans la zone prévue pour l'insertion de la canule et de préférence dans toute la région de la chambre de pression (13, 23, 33, 43, 53).
